(19) Europäisches Patentamt
European Patent Office
Office européen des brevets

(11) Publication number: **0 068 806**
**A2**

(12) **EUROPEAN PATENT APPLICATION**

(21) Application number: **82303267.7**

(22) Date of filing: **23.06.82**

(51) Int. Cl.³: **C 07 D 231/40**, C 07 D 401/12, C 07 D 403/12, C 07 D 405/12, C 07 D 409/06, C 07 D 471/04, C 07 D 453/06, C 07 D 471/10, C 07 D 491/113, A 61 K 31/415 // C07D211/14, C07D231/38 ,(C07D471/04, 231/00, 221/00),(C07D471/10, 235/00, 221/00),(C07D491/113, 327/00, 221/00)

(30) Priority: **26.06.81 US 277577**

(43) Date of publication of application: **05.01.83**
**Bulletin 83/1**

(84) Designated Contracting States: **AT BE CH DE FR GB IT LI LU NL SE**

(71) Applicant: **WARNER-LAMBERT COMPANY, 201 Tabor Road, Morris Plains New Jersey 07950 (US)**

(72) Inventor: **Dewald, Horace Albert, 1265 Bardstown, Ann Arbor Michigan 48105 (US)**
Inventor: **Lustgarten, David M., 1724 Arbordak, Ann Arbor Michigan 48105 (US)**
Inventor: **Wise, Lawrence David, 1241 Barister, Ann Arbor Michigan 48105 (US)**

(74) Representative: **Jones, Michael Raymond et al, Haseltine Lake & Co. 28 Southampton Buildings Chancery Lane, London WC2A 1AT (GB)**

(54) **Basic acyl amides of (5-amino-1,3-dialkylpyrazol-4-yl)(aryl)methanones, processes for their production, and pharmaceutical compositions containing such compounds.**

(57) Basic acylamides of (5-amino-1,3-dialkylpyrazol-4-yl)-(aryl)methanones and their pharmaceutically acceptable salts are provided, which have the formula:

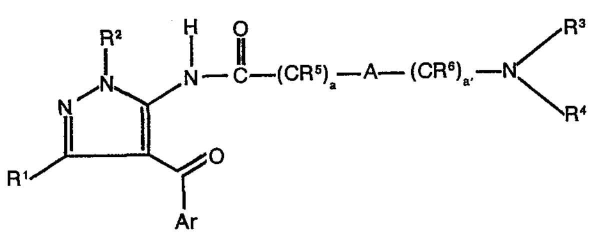

where the radicals are as defined in the specification. These compounds are useful for treating psychoses. Also provided are processes for preparing the compounds of the invention, and pharmaceutical compositions containing these compounds.

EP 0 068 806 A2

0068806



BASIC ACYL AMIDES OF (5-AMINO-1,3-DIALKYLPYRAZOL-4-YL)(ARYL)METHANONES, PROCESSES FOR THEIR PRODUCTION, AND PHARMACEUTICAL COMPOSITIONS CONTAINING SUCH COMPOUNDS

This invention relates to basic acyl amides of (5-amino-1,3-dialkylpyrazol-4-yl)(aryl)methanones, to processes for their production and to pharmaceutical compositions containing such compounds.

British Patent No. 1,337,315 discloses certain 4-benzoyl-5-aminopyrazole compounds and describes them as being useful as anti-inflammatory agents, analgesic agents and muscle relaxants.

United States Patent No. 3,558,605 discloses certain 4-aroyl-5-amino-pyrazoles which are useful as intermediates for preparing pyrazolo[3,4-e] [1,4]diazepin-7-(1H)-ones.

United States Patent No. 3,660,425 discloses certain 4-aroyl-5-amino-pyrazoles and describes them as being useful as central nervous system depressants and as chemical intermediates.

The present invention provides basic acylamides of (5-amino-1,3-dialkyl-pyrazol-4-yl)(aryl)methanones and the pharamceutically acceptable salts thereof which are useful for treating psychoses, as well as processes for their production.

One aspect of the present invention provides a compound having the following structural formula:

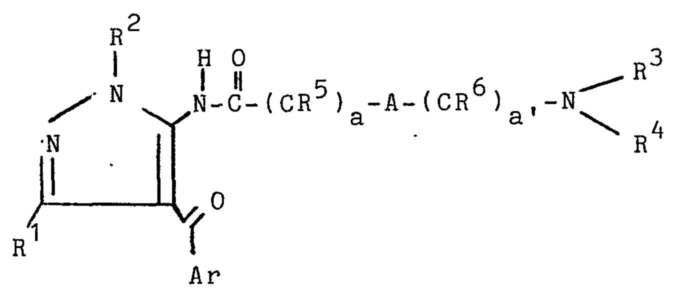

(I)

wherein each of $R^1$, $R^5$ and $R^6$, which are the same or different, is a hydrogen atom or an alkyl radical of from 1 to 6 carbon atoms; $R^2$ is an alkyl radical of from 1 to 6 carbon atoms; $R^3$ is a hydrogen atom, an alkyl radical of from 1 to 6 carbon atoms, a cycloalkyl radical of from 3 to 12 carbon atoms, or an aryl or alkaryl radical; and $R^4$ is a hydrogen atom, an alkyl radical of from 1 to 6 carbon atoms, a cycloalkyl radical of 3 to 12 carbon atoms, an aryl or alkaryl radical, a cycloalkene radical of from 5 to 7 carbon atoms which is optionally substituted with 1 or 2 alkyl radicals of from 1 to 3 carbon atoms, or a cycloalkenone radical of from 5 to 7 carbon atoms which is optionally substituted with 1 or 2 alkyl radicals of from 1 to 3 carbon atoms; or $R^3$ and $R^4$, when taken together with the nitrogen atom to which they are attached, either form a dihydropyridine, tetrahydropyridine, piperazine, piperidine, pyrrolidine or morpholine ring which is optionally substituted with 1 or 2 alkyl radicals of from 1 to 6 carbon atoms, a phenyl radical or a benzyl radical, or form one of the following substitutents:

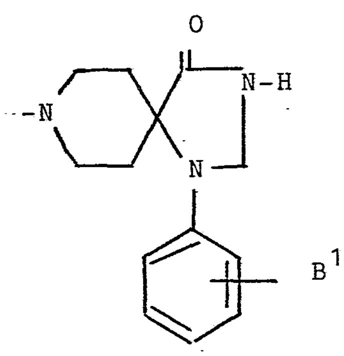

where $B^1$ is a hydrogen or halogen atom or a lower alkyl or lower alkoxy radical;

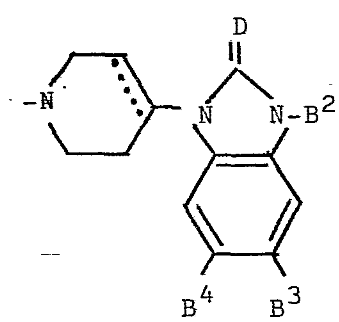

where the dotted line indicates an optional double bond; D is an oxygen or sulphur atom; $B^2$ is a hydrogen atom or a lower alkyl radical; and each of $B^3$ and $B^4$, which are the same or different, is a hydrogen or halogen atom or a lower alkyl, lower alkoxy or trifluoromethyl radical;

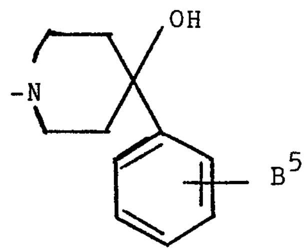

where $B^5$ is a hydrogen or halogen atom or a lower alkyl or trifluoromethyl radical;

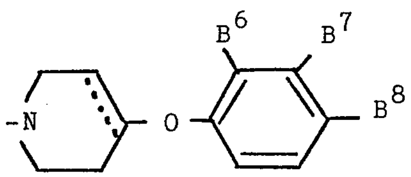

where the dotted line indicates an optional double bond; and each of $B^6$, $B^7$ and $B^8$, which are the same or different, is a hydrogen or halogen atom or a lower alkyl, lower alkoxy or trifluoromethyl radical;

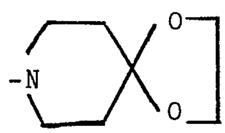

; or

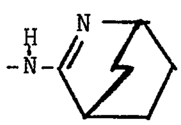

;

Ar is a phenyl radical; a phenyl radical substituted with a halogen atom, an alkyl radical of from 1 to 3 carbon atoms, an alkoxy radical of from 1 to 3 carbon atoms, or a trifluoromethyl radical; 2-, 3-, or 4-pyridyl or 2- or 3-thienyl; A is a bond or the group $OCH_2CH_2$, $SCH_2CH_2$ or $NR^7$ wherein $R^7$ is a hydrogen atom or an alkyl radical of from 1 to 6 carbon atoms; and each of a and a', which are the same or different, is zero or an integer of 1 to 6, provided that, when a is 1, $R^5$ must be a hydrogen atom; or a pharmaceutically acceptable salt thereof.

Another aspect of the present invention provides a compound having the following structural formula:

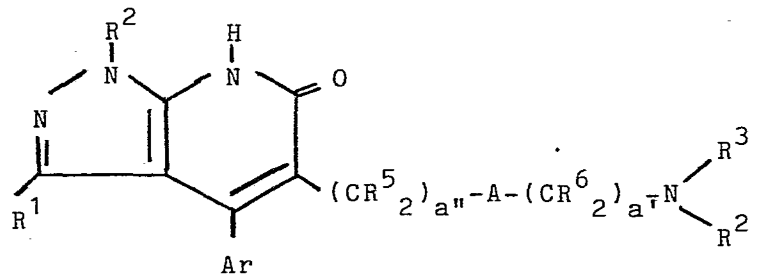

(I')

wherein $R^1$, $R^2$, $R^3$, $R^4$, $R^5$, $R^6$, Ar, A and a' are as defined above, and a" is zero or an integer of 1 to 5, or a pharmaceutically acceptable salt thereof.

A further aspect of the present invention provides a process for preparing a compound having structural formula I defined hereinabove or a salt thereof, which process comprises reacting a compound having the following structural formula IV

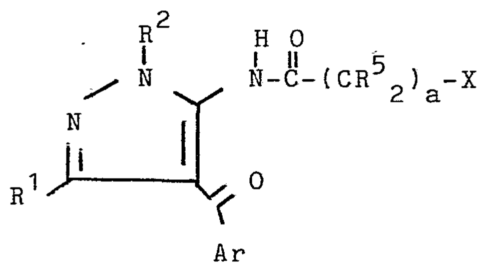

(IV)

with a compound having the following structural formula V

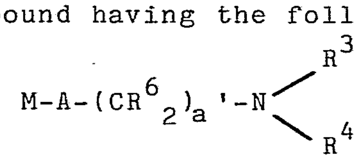

(V)

wherein $R^1$, $R^2$, $R^3$, $R^4$, $R^5$, $R^6$, Ar, A, a and a' are as defined hereinabove, M is a hydrogen atom or an alkali metal atom, and X is a halogen atom; and, if necessary, converting the reaction product to a pharmaceutically acceptable salt.

A yet further aspect of the process of the present invention is a process for producing a compound having structural formula I defined hereinabove or a salt thereof, which process comprises reacting a compound having the following structural formula IX

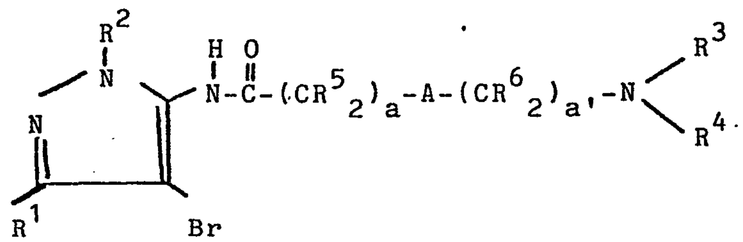 (IX)

with a compound having the following structural formula X

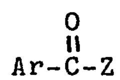 (X)

wherein $R^1$ , $R^2$, $R^3$, $R^4$, $R^5$, $R^6$, Ar, A, a and a' are defined hereinabove and Z is a halogen atom, an alkoxy radical or an equivalent thereof capable of reacting with the bromine atom; and, if necessary, converting the reaction product to a pharmaceutically acceptable salt.

An even further aspect of the present invention provides a process for producing a compound having structural formula I defined hereinabove or a salt thereof, wherein the carbon atom alpha to the amide carbonyl group is a methylene (i.e. $CH_2$) radical, which process comprises treating with a base a compound having structural formula I' given above, where $R^1$, $R^2$, $R^3$, $R^4$, $R^5$, $R^6$, Ar, A, a' and a" are as defined above; and, if necessary, converting the product to a pharmaceutically acceptable salt.

The present invention also provides, as a further aspect, a pharmaceutical composition useful for treating psychoses, which composition comprises one or more compound having the structural formula I or a pharmaceutically acceptable salt thereof, in combination with a pharmaceutically acceptable carrier.

The compounds of the present invention may be prepared by any one of several convenient procedures using starting materials which are known in the art, or are obvious variations thereof.

In a preferred process for producing the compounds of the invention, a 5-amino-4-aroylpyrazole compound having the following structural formula II

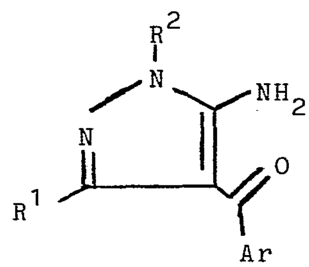

(II)

where $R^1$, $R^2$, and Ar are as defined above, is reacted with an acylating compound having the following structural formula III

$$Y-\overset{O}{\overset{\|}{C}}-(CR^5{}_2)_a-X \qquad \text{(III)}$$

where $R^5$ and a are as defined above, X is a leaving group such as a halogen, and Y is an atom or group capable of reacting with an amino group, to give the corresponding 5-acylamino-pyrazole having the following generic structural formula IV

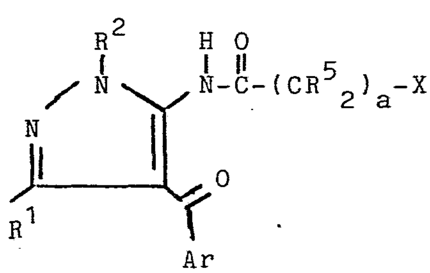

(IV)

wherein $R^1$, $R^2$, $R^5$, Ar, a and X are as defined above.

The symbols X and Y in the acylating compound of formula III both preferably represent halogen, of which bromine is preferred. X may also represent other groups

which are known to those skilled in the art as "leaving groups". The reaction between compounds II and III is generally carried out in a non-reactive solvent. When Y is a halogen atom, there is preferably a hydrogen halide acceptor such as a tertiary amine, pyridine, or an inorganic base such as sodium bicarbonate. The reactants are generally in a 1:1 ratio but an excess of compound III is acceptable. Suitable solvents are the halogenated hydrocarbons, aromatic hydrocarbons and ethers. Preferred solvents are chloroform, benzene, tetrahydrofuran, dioxane and mixtures thereof. The reaction may conveniently be performed between 0 and 100°C and will generally be complete within 24 hours; it has generally been observed that the reaction is substantially complete within from 2-5 hours when a temperature of from 60 - 80°C is utilized. The requisite starting materials of structural formula II may be prepared as described in United States Patent No. 3,660,425 and in British Patent No. 1,337,315. The acylating compounds of structural formula III are either commercially available or may be prepared by procedures well known to those skilled in the art.

The compounds of structural formula IV are next reacted with a compound of the following structural formula V

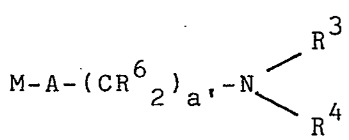

(IV)

where $R^3$, $R^4$, $R^6$, A and a' are as defined above, thereby producing the compounds of the invention of structural formula I. An excess of compound V is preferably utilized. The symbol M in structural formula V represents hydrogen or an alkali metal. Thus, when A represents $NR^7$, M is preferably hydrogen; and, when A represents $OCH_2CH_2$ or $SCH_2CH_2$, M is preferably sodium. The reaction between compounds IV and V is generally carried out in a non-reactive solvent such as an aliphatic or aromatic hydrocarbon, an ether, a lower alcohol or mixtures thereof. When M represents hydrogen, halogenated hydrocarbon solvents may also be used. Preferred solvents are hexane, benzene, tetrahydrofuran, dioxane and chloroform. The reaction may conveniently be carried out at from -10 to 100°C and is generally complete within 30 hours. At temperatures of from 20 to 60°C the reaction has been observed to be substantially complete within about 5 to 10 hours. In a reaction where a hydrogen halide will be generated, e.g. where M is hydrogen and X is bromine, an acid acceptor such as a tertiary amine, pyridine, or an inorganic base such as sodium bicarbonate will preferably be present.

In a second process for preparing the compounds of structural formula I, a 5-aminopyrazole having the following structural formula VI

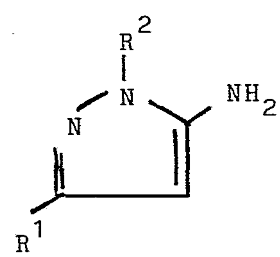

(VI)

where $R^1$ and $R^2$ are as defined above, is reacted with an acylating compound having the structural formula III defined above. The reaction between compounds III and VI is carried out utilizing substantially the same reaction conditions as already described for the reaction between compounds II and III. The compounds of structural formula VI may be prepared as described in United States Patent No. 3,660,425.

The products of the reaction between compounds III and VI have the following generic structural formula VII

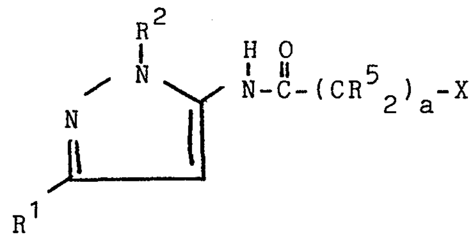

(VII)

where $R^1$, $R^2$, $R^5$, X and a are as defined above, and are next reacted with a compound having the structural formula V as defined above, utilizing substantially the same reaction conditions already described for the reaction between compounds IV and V.

The products of the reaction between compounds V and VII have the following generic structural formula VIII

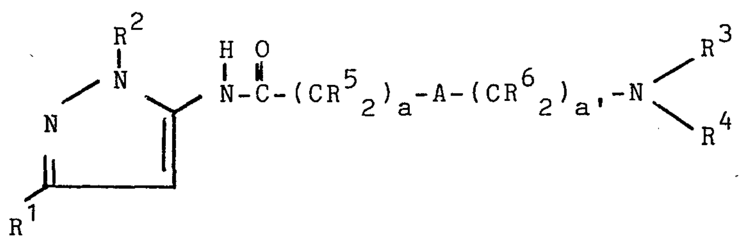

(VIII)

where $R^1$, $R^2$, $R^3$, $R^4$, $R^5$, $R^6$, A, a and a' are as defined above, and are next brominated using an equivalent quantity or a slight excess of bromine to produce a compound having the following generic structural formula IX

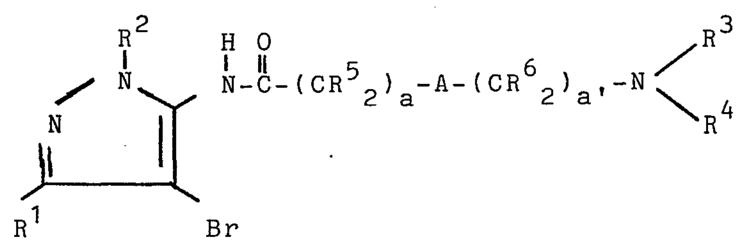

(IX)

The bromination is generally carried out in a non-reactive solvent such as unreactive halogenated hydrocarbon, aromatic

hydrocarbon, ether, lower alcohol, lower alkanoic acid or mixtures thereof. Preferred solvents are chloroform, benzene, tetrahydrofuran, dioxane, methanol, ethanol and acetic acid. The reaction is carried out at temperatures of from -10 to 50$^{o}$C, and is substantially complete within 5 hours.

The compound of formula IX is then treated in a two step reaction sequence to produce the final products of structural formula I. In the first step the compound of structural formula IX is metalated with, for example, a compound such as butyl lithium and the so obtained lithium derivative is then reacted in the second step with a compound of structural formula X

$$Ar-\overset{O}{\overset{\|}{C}}-Z \qquad (X)$$

Symbol Z in structural formula X represents a halogen atom, alkoxy radical of from 1 to 6 carbon atoms or an equivalent group known to those skilled in the art. For purposes of the invention, Z is preferably chlorine. The lithiation is conveniently carried out under anhydrous conditions in a reaction-inert solvent such an an aliphatic hydrocarbon, an aromatic hydrocarbon, an ether or mixtures thereof. Preferred solvents are tetrahydrofuran, diethylether and hexane. A minimum of 2 equivalents of the lithiating agent is utilized and an additional equivalent is used for each "active" hydrogen present in the compound of structural formula IX. For purposes of this reaction, those skilled in the art will understand what is meant by the term "active" hydrogen, an example of which is a hydrogen bonded to a nitrogen atom. The lithium derivative is then treated with one equivalent of a compound of structural formula X. Both steps of this reaction sequence are carried out at a temperature of from -60$^{o}$C to -0$^{o}$C, preferably -60$^{o}$C, and each step requires from about 1 to about 6 hours.

Certain of the compounds of the present invention, of structural formula I, may exist in equilibrium with a cyclized structure which has eliminated the elements

of water. The compounds of formula I which may participate in this equilibrium are limited to those which possess a methylene group (i.e. $CH_2$) in the position alpha to the amide carbonyl group and are described by the following partial structural formula

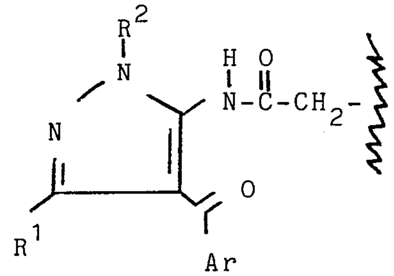

For purposes of the invention these forms are referred to herein as the "open-chain" form and the "cyclized" form. Thus, for example, this equilibrium has been observed for the following compound of the present invention:-

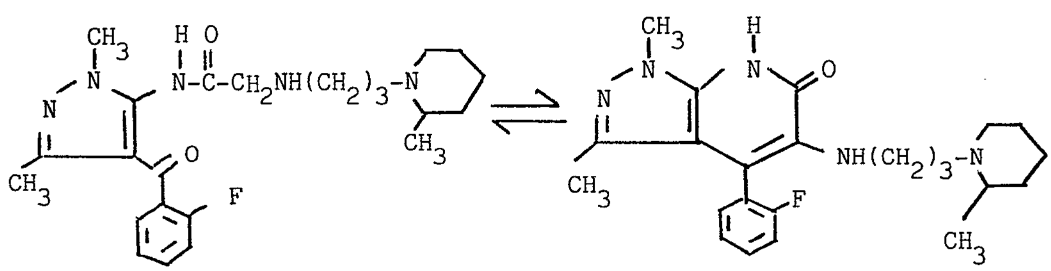

open chain cyclized

The "cyclized" form has been isolated during the synthesis of certain of the compounds of formula I, thus indicating that the "cyclized" form may be involved as an intermediate in the synthetic processes of the present invention.

The compounds of the present invention form pharmaceutically acceptable salts with organic and inorganic acids.

and alkaline earth bases. Examples of suitable acids for salt formation are hydrochloric, sulfuric, phosphoric, acetic, citric, oxalic, malonic, salicylic, malic, fumaric, succinic, malonic, ascorbic, maleic, methanesulfonic and the like. The salts are prepared by contacting the free base form with an equivalent amount of the desired acid base in the conventional manner. The free base forms may be regenerated by treating the salt form with a base. For example, dilute aqueous base solutions may be utilized. Dilute aqueous sodium hydroxide, potassium carbonate, ammonia and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention.

Examples of suitable bases for salt formation are sodium hydroxide, potassium hydroxide, calcium hydroxide, and the like. The salts are prepared by contacting the parent compound with an equivalent amount of the desired base in the conventional manner. The parent compound may be regenerated by treating the salt form with an acid. For example, dilute aqueous acid solutions may be utilized. Dilute aqueous hydrochloric acid, sulfuric acid or acetic acid are suitable for this purpose. The parent forms differ from their respective salt forms somewhat in certain physical properties such as solubility in polar solvents, but the salts are otherwise equivalent to their respective parent forms for purposes of the invention.

The compounds of the invention can exist in unsolvated as well as solvated forms, including hydrated forms. In general, the solvated forms, with pharmaceutically acceptable solvents such as water, ethanol and the like are equivalent to the unsolvated forms for purposes of the invention.

Selected compounds of the invention may exist in optical or in geometric isomeric forms. For purposes of the invention, the individual pure isomers as well as mixtures thereof are considered to be equivalent.

The alkyl and alkoxy groups contemplated by the invention, unless specified otherwise, comprise both straight and branched carbon chains of from 1 to about 6 carbon atoms. Representative of such groups are methyl, ethyl, isopropyl, butyl, pentyl, 3-methylpentyl, methoxy, ethoxy, *i*-propoxy, *t*-butoxy, *n*-hexoxy, 3-methylpentoxy and the like.

The term cycloalkyl when used herein is intended to include monocylic, bicyclic and spirocyclic carbocyclic rings and ring system of from 3 to 12 carbon atoms. Representative of such groups are cyclopropyl, cyclopentyl, cyclohexyl, norbornyl, adamantyl, spiro[3,3]heptyl and the like. These rings and ring systems may in addition be substituted by up to 2 alkyl groups of from 1 to 3 carbon atoms.

The term aryl when used herein is intended to include phenyl; 2-,3-,4-pyridyl; and 2-,3-thienyl.

The term alkaryl when used herein is intended to include $-(CH_2)_{1-3}$-aryl wherein aryl is defined in the preceding paragraph.

The terms cycloalkene and cycloalkenone when used herein are intended to include both monocyclic and bicyclic ring systems. Examples of contemplated groups are cyclopentanone, cyclohexanone, norbornanone, cyclopentene, cyclohexene, norbornene and the like. The cycloalkenes and cycloalkenones may, in addition, be substituted by up to 2 alkyl groups of from 1 to 3 carbon atoms. Also contemplated are monocyclic and bicyclic cycloalkenones such as cyclopentenone, cyclohexenone and norbornenone. The carbonyl groups of the contemplated ketones may also be in the form of well known derivatives such as an ethylene ketal, an oxime, an oxime ester and the like.

The term halogen unless specified otherwise is intended to include fluorine, chlorine, bromine and iodine.

The compounds of the invention are new chemical substances which are useful as pharmaceutical agents for the treatment of psychoses. The anti-psychotic activity of representative compounds of the invention was established by the Mouse Activity

and Screen Test Procedure (MAST) described below. Animals. Nine unfasted Swiss-Webster male mice (Buckberg Labs) weighing 20-30 g are equally divided into three groups for each drug dose to be tested. That is, data for each dose level was generated by 3 separate groups of 3 mice each. Drugs.- A minimum of three dose levels (10, 30, and 100 mg/kg) are tested for each drug. Treatments are administered intraperitoneally one hour prior to testing. All dosages are calculated as parent compound and given in volumes of 10 ml/kg. Compounds are dissolved or suspended in 0.2% Methocel. Control animals are injected with Methocel.

TESTING: A two part testing procedure is started one hour post-injection. First, the screen test is performed (see Pharmac. Biochem. Behav. 6, 351-353, 1977). Briefly this test consists of placing mice on individual wire screens which are then rotated 180 degrees at the start of a 60 second observation period. The number of mice falling off the inverted screen is recorded.

Immediately following the screen test, the final phase of testing is initiated by placing each group of 3 mice in one actophotometer (Life Sciences, 22, 1067-1076, 1978). The actophotometer consists of a cylindrical chamber whose center is occupied by another cylinder which contains the illumination for 6 photocells located on the perimeter of the chamber. Six light-beam interruptions equal one count. Locomotor activity is recorded by computer at 10 minute intervals for 60 minutes.

DATA: The data obtained from the screen test are expressed as percent of mice falling off the screen. Data derived from locomotor activity of drug treated mice are compared to the activity of vehicle treated animals and are expressed as percent inhibition of spontaneous locomotion. All percentages reported for inhibition of locomotion are based upon data accumulate for one hour. Both phases of testing are graded: A=60-100%; C=31-59%; and N=0-30%. An overall dose rating is obtained by the following criteria:

| Inhibition of Locomotion Rating | with | Screen Test Failure Rating | = | Dose Rating |
|---|---|---|---|---|
| A | - | N or C | = | A |
| A | - | A | = | C |
| C | - | N or C | = | C |
| | | All other combinations | = | N |

Compounds which exhibit an overall dose rating of A at a dose of 100 milligrams/kilogram or less are considered active. Utilizing this procedure, an overall dose rating of A was obtained for the noted compound at the indicated dose. The compounds are identified in the Examples.

TABLE I

| Compound | Dose (mg/kg) |
|---|---|
| 1 | 100; 30 |
| 2 | 100 |
| 3 | 100 |
| 4 | 100; 30 |
| 5 | 100; 30 |
| 6 | 100; 30 |
| 7 | 100 |
| 8 | 100; 30 |
| 9 | 100; 30 |
| 10 | 100; 30 |
| 11 | 100; 30 |
| 12 | 100 |
| 13 | 100 |
| 14 | --- |
| 15 | 100 |
| 16 | 100 |
| 17 | 100 |
| 18 | 100 |
| 19 | 100 |
| 20 | 100; 30 |
| 21 | 100 |
| 22 | 30 |
| 23 | 100 |
| 24 | 100 |
| 25 | 100 |
| 26 | 100 |

TABLE I
(Continued)

| Compound | Dose (mg/kg) |
|---|---|
| 27 | 100 |
| 28 | --- |
| 29 | 100 |
| 30 | 100 |
| 31 | 100 |
| 32 | 100 |
| 33 | 100; 30 |
| 34 | 100; 30 |
| 35 | 100; 30 |
| 36 | 100 |
| 37 | 100; 30 |
| 38 | 100 |
| 39 | 100 |
| 40 | 100 |
| 41 | 100; 30 |
| 42 | 100; 30 |
| 43 | 100; 30 |
| 44 | 100; 30 |
| Sulpiride | 100 |
| Clozapine | 10 |
| Thioridazine | 45; 15 |

Representative compounds of the invention (identified in the examples) were also tested for antipsychotic activity according to the following protocol. The noted compounds had the indicated $ED_{50}$ values (mg/kg) and are considered active as antipsychotic agents in the test procedure.

PROCEDURE:

Mature male Long-Evans rats or squirrel-monkeys are conditioned to push a lever in order to avoid a painful electric footshock. If the animal fails to push the lever, he receives a shock every 10 seconds until the lever is pushed. Shocks can be terminated by pushing the lever. Thereafter, as long as the lever is pushed at least once every 20 seconds, there will be no shock.

Each animal acts as its own control; one weekly session is used to establish baseline behavior and another session later in the week is used as a drug session. Once patterns of avoidance are established, the effects of standard and unknown compounds are studied.

RESPONSE EVALUATION

All events are electronically programmed and the response to these events counted or used as feed-back to the program.

| Compound | $ED_{50}$ (mg/kg) |
|---|---|
| 1 | 48.2 |
| 5 | 48.0 |
| 6 | 25.6 |
| 8 | 36.4 |
| 9 | 36.3 |
| 20 | 21.4 |
| 35 | 51.6 |
| 38 | 21.7 |
| 43 | 26.5 |

The compounds of the invention can be prepared and administered in a wide variety of oral parenteral dosage forms. It will be clear to those skilled in the art that the following dosage forms may comprise as the active component, either a compound of formula I, or a corresponding pharmaceutically acceptable salt of a compound of formula I, or a mixture or such compounds and/or salts.

For preparing pharmaceutical compositions from the compounds described by this invention, inert, pharmaceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the

active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, i.e., natural or synthetic gums, resins, methyl cellulose, sodium carboxymethyl cellulose and other well-known suspending agents.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example,

packeted tablets, capsules and powders in vials or ampoules. The unit dosage form can also be a capsule, cachet or tablet itself or it can be the appropriate number of any of these packaged form.

The quantity of active compound in a unit dose of preparation may be varied or adjusted from 1 mg to 100 mg according to the particular application and the potency of the active ingredient.

In therapeutic use as agents for treating psychoses in mammals, the compounds utilized in the pharmaceutical method of this invention are administered at the initial dosage of about 0.1 mg to about 100 mg per kilogram daily. A daily dose range of about 1.0 mg to about 50 mg per kilogram is preferred. The dosages, however, may be varied depending upon the requirements of the patient, the severity of the condition being treated and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following non-limiting examples illustrate the inventors' preferred methods for preparing the compounds of the invention.

EXAMPLE I

N-[4-(2-Fluorobenzoyl)-1,3-dimethyl-1H-pyrazol-5-yl]-1-pyrrolidineacetamide, (Compound 1), a suspension of 14 g (0.04 mol) of N-[4-(2-fluorobenzoyl)-1,3-dimethyl-1H-pyrazol-5-yl]-2-bromoacetamide in 180 ml of dichloromethane is stirred and cooled at 10-15°C as 7 ml (0.05 mol) of triethylamine and then 6 g (0.08 mol) of pyrrolidine is added dropwise. A pale yellow solution results after a short time and the solution is allowed to stand at room temperature for 16 hours. The reaction mixture is washed with a saturated aqueous solution of $NaHCO_3$, dried over $MgSO_4$ and evaporated in vacuo. The residue (12 g) is crystallized from ether-petroleum ether to yield 11.1 g (81%) of product, mp. 116-118°C. The hydrochloride salt is prepared by dissolving the base in

tetrahydrofuran, treating with excess 20% isopropanolic·HCl and diluting with ether to turbidity, mp 180-183°C dec. In a similar manner, but substituting N-[4-(3 or 4-fluorbenzoyl)-1,3-dimethyl-1H-pyrazol-5-yl]-2-bromoacetamide there is obtained N-[4-(3-fluorobenzoyl)-1,3-dimethyl-1H-pyrazol-5-yl]-1-pyrrolidineacetamide (Compound 2), mp 115-117°C, recrystallized from ethyl acetate/petroleum ether; and N-[4-(4-fluorobenzoyl)-1,3-dimethyl-1H-pyrazol-5-yl]-1-pyrrolidineacetamide (Compound 3), mp 167-169°C recrystallized from ethyl acetate/petroleum ether.

By the same procedure and using the indicated moles of N-[4-(2-fluorobenzoyl)-1,3-dimethyl-1H-pyrazol-5-yl]-2-bromoacetamide (Compound "a") and the desired amine, in place of pyrrolidine the following compounds are obtained:

N-[4-(2-fluorobenzoyl)-1,3-dimethyl-1H-pyrazol-5-yl]-2,5-dimethyl-1-pyrrolidineacetamide, (Compound 4), mp 82-84°C from 3.2 g (0.009 mol) of (a) and 2 g (0.02 mol) of 2,5-dimethylpyrrolidine and 3 ml $Et_3N$.

N-[4-(2-Fluorobenzoyl)-1,3-dimethyl-1H-pyrazol-5-yl]-1-piperidineacetamide, (Compound 5), mp 138-140°C from 5.3 g (0.015 mol) of (a) and 3 ml of piperidine and 3 ml $Et_3N$.

2-(Diethylamino)-N-[4-(2-fluorobenzoyl)-1,3-dimethyl-1H-pyrazol-5-yl]acetamide, (Compound 6), from 5.3 g (0.015 mol) of (a) and 4 ml of diethylamine. The dihydrochloride salt melts at 170-173°C dec.

N-[4-(2-Fluorobenzoyl)-1,3-dimethyl-1H-pyrazol-5-yl]-2-methyl-1-aziridineacetamide, (Compound 7), from 2.5 g (0.007 mol) of (a) and 2 ml of 2-methylaziridine, mp of the dihydrochloride salt 185°C.

N-[4-(2-Fluorobenzoyl)-1,3-dimethyl-1H-pyrazol-5-yl]-3-[[(2-methyl-1,3-dioxolan-2-yl)methyl]amino]acetamide, (Compound 8), mp 165-167°C (as the dihydrochloride salt) from 3.6 g (0.01 mol) of (a) and 1.3 g of aminoacetone ethyleneketal.

N-[4-(2-Fluorobenzoyl)-1,3-dimethyl-1H-pyrazol-5-yl]-3-azabicyclo-[3,2,2]nonane-3-acetamide, (Compound 9), mp 265-270°C (as the hydrochloride salt) from 4.4 g (0.012 mol) of (a) and 2.1 g (0.017 mol) of 3-azabicyclo[3,2,2]nonane.

N-[4-(2-Fluorobenzoyl)-1,3-dimethyl-1H-pyrazol-5-yl]-2-(bicyclo-[2,2,1]hept-2-ylamino)acetamide, (Compound 10), mp 193-195°C (as the dihydrochloride salt) from 3.6 g (0.01 mol) of (a) and 1.5 g (0.01 mol) of endo-2-aminonorbornane.HCl and 4 ml of triethylamine.

N-[4-(2-Fluorobenzoyl)-1,3-dimethyl-1H-pyrazol-5-yl]-2-[[3-(2-oxo-1-pyrrolidinyl)propyl]amino]acetamide, (Compound 11), mp 150-152° (as the maleate salt) from 7.08 g (0.02 mol) of (a) and 5.80 g of 3-(2-oxo-1-pyrrolidinyl)propylamine.

N-[4-(2-Fluorobenzoyl)-1,3-dimethyl-1H-pyrazol-5-yl]-2-(tricyclo-[3.3.1.13,7]dec-1-yl)amino]acetamide, (Compound 12), mp 215-218° (as the monohydrochloride salt, dihydrate) from 3.7 g (0.01 mol) of (a) and 1.9 g of tricyclo[3.3.1.13,7]dec-1-ylamine.

N-[4-(2-Fluorobenzoyl-1,3-dimethyl-1H-pyrazol-5-yl]-4-oxo-1-phenyl-1,3,8-triazaspiro[4,5]decan-8-acetamide, (Compound 13), mp 145-147°C from 2.2 g (.0062 mol) of (a) and 1.8 g of 1-phenyl-1,3,8-triazaspiro[4,5]decan-4-one and 1.5 ml of triethylamine.

N-(4-Benzoyl-1,3-dimethyl-1H-pyrazol-5-yl)-2-(diethylamino)-acetamide, (Compound 14), mp 120°C (as the dihydrochloride salt, monohydrate) from 3.2 g (0.0075 mol) of N-[4-(benzoyl)-1,3-dimethyl-1H-pyrazol-5-yl]-2-bromoacetamide (U.S. Patent No. 3,558,605) and 4 ml of diethylamine.

N-[4-(2-Chlorobenzoyl)-1,3-dimethyl-1H-pyrazol-5-yl]-2-(diethyl-amino)acetamide, (Compound 15), mp 157-161°C (as the dihydro chloride salt, hemihydrate) from 3.7 g (0.01 mol) of N-[4-(2-chlorobenzoyl)-1,3-dimethyl-1H-pyrazol-5-yl]-2-bromoacetamide (U.S. Patent No. 3,558,605) and 4 ml of diethylamine.

2-(Diethylamino)-N-[1,3-dimethyl-4-(3-thienylcarbonyl)-1H-pyrazol-5-yl]acetamide, (Compound 16), mp 147-150°C (as the dihydrochloride salt, monohydrate) from 3.0 g (0.01 mol) of 2-chloro-N-[1,3-dimethyl-4-(3-thienylcarbonyl)-1H-pyrazol-5-yl]-acetamide and 4 ml of dietylamine. The intermediate chloroacetamide is prepared by refluxing 10 g (0.045 mol) of (5-amino-1,3-dimethyl-pyrazol-4-yl)-3-thienylmethanone with 4.8 g (0.045 mol) of chloroacetyl chloride in 100 ml of dichloroethane for 1 hr. The cooled reaction mixture is stirred with 75 ml of a saturated aqueous solution of $NaHCO_3$ and filtered to yield 8.2 g, mp 167-169°C.

2-(Diethylamino)-N-[1,3-dimethyl-4-(2-thienylcarbonyl)-1H-pyrazol-5-yl]acetamide, (Compound 17), mp 145°C dec (as the dihydrochloride salt, monohydrate) from 3.4 g (0.01 mol) 2-bromo-N-[1,3-dimethyl-4-(3-thienylcarbonyl)-1H-pyrazol-5-yl]acetamide and 4 ml of diethylamine. The requisite bromoacetamide is prepared by treating (5-amino-1,3-dimethylpyrazol-4-yl)-2-thienylmethanone (U.S. Patent No. 3,660,425) with an equivalent of bromoacetyl bromide in refluxing dichloroethane. The 2-bromo-N-[1,3-dimethyl-4-(2thienylcarbonyl)-1H-pyrazol-5-yl]-acetamide melts at 165-168°C from acetonitrile-ether.

By the procedure of example I, but using 4.4 g (0.014 mol) of N-[4-(2-fluorobenzoyl)-1,3-dimethyl-1H-pyrazol-5-yl]-3-chloropropanamide, mp 164-166°C, in place of compound (a) and 2 ml of pyrrolidine and 2 ml of $Et_3N$, there is obtained N-[4-(2-fluorobenzoyl)-1,3-dimethyl-1H-pyrazol-5-yl]-1-pyrrolidinepropanamide, (Compound 18), mp 108-110°C.

By using 5.5 g (0.017 mol) of N-[4-(2-fluorobenzoyl)-1,3-dimethyl-1H-pyrazol-5-yl]-3-chloropropionamide and 3.3 g (0.03 mol) of aminoacetone ethyleneketal in the presence of 3 ml $Et_3N$, one obtains N-[4-(2-fluorobenzoyl)-1,3-dimethyl-1H-pyrazol-5-yl]-3-[[2-methyl-1,3-dioxolan-2-yl)methyl]amino]propanamide, (Compound 19), mp of the maleate salt, 152-154°C.

## EXAMPLE II

N-[4-(2-Fluorobenzoyl)-1,3-dimethyl-1H-pyrazol-5-yl]-2-[[3-(2-methyl-1-piperidinyl)propyl]amino]acetamide, (Compound 20), a solution of 17.7 g (0.05 mol) of (a) in 1 liter of toluene at 60°C is treated in one portion with 16.3 g (0.1 mol) of 1-(3-amino propyl)-2-pipecoline and stirred at 60-80°C for 2 hours. The toluene solution is decanted from some gum and washed with 1-L of 5% $NaHCO_3$ solution, then dried over $MgSO_4$. An ether solution of 11.6 g (0.1 mol) of maleic acid is added and the collected precipitate is recrystallized by dissolving in acetonitrile (250 ml) and adding ethyl acetate (300 ml) to give 16 g (50%) mp 148-150°C of the dimaleate salt of the title compound. By using the same procedure and using the indicated moles of compound (a) and the desired amine, the following compounds are obtained:

2-[3-(Dimethylamino)propyl]-N-[4-(2-fluorobenzoyl)-1,3-dimethyl-1H-pyrazol-5-yl]acetamide, (Compound 21), from 7 g (0.02 mol) compound (a) and 4.1 g (0.04 mol) of 3-dimethylaminopropylamine and isolated as the dimaleate salt, mp 136-139°C.

N-[4-(2-Fluorobenzoyl)-1,3-dimethyl-1H-pyrazol-5-yl-2-[[3-(2,6-dimethyl-1-piperidinyl)propyl]amino]acetamide, (Compound 22), from 5.0 g (0.013 mol) compound (a) and 4.7 g (0.028 mol) of 1-(3-aminopropyl)-2,6-dimethylpiperidine, characterized as the oxalate salt mp 187°C.

4-(2-Chlorophenyl)-N-[4-(2-fluorobenzoyl)[1,3-dimethyl-1H-pyrazol-5-yl]-1-piperazineacetamide, (Compound 23), mp 137-138°C, (as the dimaleate salt) from 2.5 g (0.007 mol) compound (a) and 3.9 g (.02 mol) 4-(2-chlorophenyl)piperazine.

4-(2-Methylphenyl)-N-[4-(2-fluorobenzoyl)[1,3-dimethyl-1H-pyrazol-5-yl]-1-piperazineacetamide, (Compound 24), mp 153-155°C (as the dimaleate salt) from 3.5 g (.01 mol) compound (a) and 3.9 g (.02 mol) 4-(2-methylphenyl)piperazine.

N-[4-(2-Fluorobenzoyl)-1,3-dimethyl-1H-pyrazol-5-yl]-2-[[2-(3,6-dihydro-4-phenyl-1(2H)-pyridinyl)ethyl]amino]acetamide, (Compound 25), mp 170-172°C (as the dimaleate salt) from 2.7 g (0.0075 mol) of compound (a) and 2.2 g (0.008 mol) of 2-(3,6-dihydro-4-phenyl-1(2H)-pyridinyl)ethylamine.

N-[4-(2-Fluorobenzoyl)-1,3-dimethyl-1H-pyrazol-5-yl]-2-[[3-(4-methyl-1-piperidinyl)propyl]amino]acetamide, (Compound 26), mp 168-170°C (as the dimaleate salt) from 3.54 g (0.010 mol) of compound (a) and 3.30 g (0.020 mol) of 3-(4-methyl-1-piperidinyl)propylamine.

N-[4-(2-Fluorobenzoyl)-1,3-dimethyl-1H-pyrazol-5-yl]-2-[[3-(3-methyl-1-piperidinyl)propyl]amino]acetamide, (Compound 27), mp 167-168°C (as the dimaleate salt) from 3.54 g (0.010 mol) of compound (a) and 3.30 g (0.020 mol) of 3-(3-methyl-1-piperidinyl)-propylamine.

EXAMPLE III

N-[4-(2-Fluorobenzoyl)-1,3-dimethyl-1H-pyrazol-5-yl]-2-[[3-(2,5-dimethyl-1-pyrrolidinyl)propyl]amino]acetamide, (Compound 28). A refluxing solution of 5.0 (0.014 mol) of (a) in 50 ml of dichloromethane is treated dropwise with 4.4 g (0.028 mol) of 1-(3-aminopropyl)-2,5-dimethyl pyrrolidine and the mixture is refluxed for 0.5 hours. The cooled solution is washed with saturated $NaHCO_3$ solution, dried over $MgSO_4$ and evaporated in vacuo. The residue, redissolved in ether, is filtered into a solution of 3.3 g (0.03 mol) of maleic acid in 400 ml of ether. The resulting precipitate is collected and recrystallized from 100 ml of acetonitrile to give 5.34 g mp 128-129.5°C of the dimaleate salt of the title compound. By using the same procedure and using the indicated moles of compound (a) and the desired amine, the following compounds are obtained:

N-[4-(2-Fluorobenzoyl)-1,3-dimethyl-1H-pyrazol-5-yl]-2-[[3-(2-methyl-1-piperidinyl)propyl]-N-methylamino]acetamide, (Compound 29), from 5.0 g (0.014 mol) compound (a) and 5 g (0.078 mol) of (N-methylaminopropyl)-2-methylpiperidine, characterized as the dimaleate salt mp 150.5-151.5°C. The requisite diamine is prepared by formylating 1-(3-aminopropyl)-2-methylpiperidine and reduction of the formamide with lithium aluminum hydride. [Other diamines, not available commercially, are prepared in a similar manner, e.g., reaction of the piperidine or pyrrolidine with chloroacetaonitrile or acrylonitrile and reduction with lithium aluminum hydride.

N-[4-(2-Fluorobenzoyl)-1,3-dimethyl-1H-pyrazol-5-yl]-2-[[3-(2-ethyl-1-piperidinyl)propyl]amino]acetamide, (Compound 30), from 5.0 g (0.014 mol) compound (a) and 4.8 g (0.03 mol) 1-(3-aminopropyl)-2-ethylpiperidine, mp 118-120°C as the dimaleate salt.

N-[4-(2-Fluorobenzoyl)-1,3-dimethyl-1H-pyrazol-5-yl]-2-[[3-(t-butylamino)propyl]amino]acetamide, (Compound 31), from 5 g (0.014 mol) of compound (a) and 3.0 g (0.02 mol) of 3-(t-butylamino)-propylamine, dimaleate salt mp 187-189°C.

N-[4-(2-Fluorobenzoyl)-1,3-dimethyl-1H-pyrazol-5-yl]-2-[[3-(1-piperidinyl)propyl]amino]acetamide, (Compound 32), from 3.54 g (0.01 mol) of compound (a) and 2.85 g (0.02 mol) of 1-(3-aminopropyl)piperidine. Compound 32 was purified as the dimaleate salt, mp 163-5°C.

N-[4-(2-Fluorobenzoyl)-1,3-dimethyl-1H-pyrazol-5-yl]-2-[[3-(3-ethyl-6-methyl-1-piperidinyl)propyl]amino]acetamide, (Compound 33), from 3.54 g (0.01 mol) of compound (a) and 3.4 g (0.02 mol) of 1-(3-aminopropyl)-3-ethyl-6-methylpiperidine and characterized as the dimaleate salt, mp 110-116°C.

N-[4-(2-Fluorobenzoyl)-1,3-dimethyl-1H-pyrazol-5-yl]-2-[[2-(1-piperidinyl)ethyl]amino]acetamide, (Compound 34), from 3.5 g (0.01 mol) of compound (a) and 2.6 g (0.02 mol) of 1-(2-aminoethyl)piperidine; characterized as the dimaleate salt, mp 154°C.

N-[4-(2-Fluorobenzoyl)-1,3-dimethyl-1H-pyrazol-5-yl]-2-[[3-(4-(morpholinyl)propyl]amino]acetamide,(Compound 35), from 3.5 g (0.01 mol) of compound (a) and 2.9 g (0.02 mol) of 1-(3-aminopropyl)morpholine; characterized as the dimaleate salt, mp 163-165°C.

N-[4-(2-Fluorobenzoyl)-1,3-dimethyl-1H-pyrazol-5-yl]-2-[[3-(4-(2-hydroxyethyl-1-piperazinyl)propyl]amino]acetamide, (Compound 36), from 3.5 g (0.01 mol) of compound (a) and 3.75 g (0.02 mol) of 3-(aminopropyl)piperazineethanol and isolated as its trimaleate salt mp 156°C.

## EXAMPLE IV

N-[4-(2-Fluorobenzoyl)-1,3-dimethyl-1H-pyrazol-5-yl]-2-[[2-(3-methyl-1-piperidinyl)ethyl]amino]acetamide, (Compound 37), a mixture of 7.08 g (0.02 mol) compound (a) and 5.68 g (0.04 mol) of 1-(2-aminoethyl)-3-methylpiperidine in 200 ml of $CH_2Cl_2$ is refluxed for 2 hours. After coooling, the mixture is washed with 5% $NaHCO_3$ solution, and evaporated in vacuo. The residue is dissolved in 250 ml of ether and extracted with 50 ml of 2 N NaOH. The alkaline extract is diluted with 50 ml of 50% NaOH to precipitate an oil which is extracted into ether. The ether solution, dried over $MgSO_4$, is treated with 3.3 g (0.03 mol) of maleic acid. The precipitate is collected and recrystallized from 50 ml acetonitrile and 125 ml ethyl acetate to yield 1.7 g., mp 110-112°C. of the title compound as the dimaleate salt.

N-[4-(2-Fluorobenzoyl)-1,3-dimethyl-1H-pyrazol-5-yl]-2-[methyl[2-[(2-methyl-3-oxo-1-cyclopenten-1-yl)amino]ethyl]amino]acetamide, (Compound 38), from the reaction of 3.6 g (0.01 mol) of compound (a) and 2.9 g (0.01 mol) of the maleate salt of 1-methyl-2-[2-methyl-3-oxo-1-cycloptenen-1-yl)amino]ethylamine in the presence of 5.4 ml (0.03 mol) of triethylamine. The title compound as the free base melts at 136-138°C. The starting amine is prepared by refluxing a solution of 5.7 g (0.05 mol) of 2-methyl-1,3-cyclopentanedione and 4 g (0.05 mol) of N-methylethylenediamine in 100 ml of toluene for 3 hours. After evaporation of the solvent, the product is isolated as the monomaleate salt by adding 5.5 g (0.05 mol) of maleic acid to a solution of the reaction product in methanol and diluting with THF-ether; mp 158-160°C.

N-[4-(2-Fluorobenzoyl)-1,3-dimethyl-1H-pyrazol-5-yl]-2-[[3-[(5,5-dimethyl-3-oxo-1-cylcohexen-1-yl)amino]propyl]methylamino]-acetamide, (Compound 39), from the reaction of 3.6 g (0.01 mol) of compound (a) and 3.3 g (0.01 mol) of the maleate salt of 5,5-dimethyl-3-[3-(methylamino)propyl]amino]-2-cyclohexen-1-one in the presence of 5.4 ml (0.03 mol) of triethylamine. The title compound as the maleate salt melts at 152-154°C, after recrystallization from THF-ethylacetate. The starting amine is prepared by refluxing a solution of 7 g (0.05 mol) of 5,5-dimethyl-1,3-cyclohexanedione and 4 g (0.05 mol) of N-methylethylenediamine in 100 ml of toluene for 3 hours. After evaporation of the solvent, the product is isolated as the monomaleate salt by adding 5.5 g (0.05 mol) of maleic acid to a solution of the reaction product in methanol and diluting with THF; mp 142-145°C.

N-[4-(2-Fluorobenzoyl)-1,3-dimethyl-1H-pyrazol-5-yl]-1-(4-fluorophenyl)spiro[3H-oxazolo[3,4-a]pyridine-3,4'-piperidine]-1'-acetamide, (Compound 40), is prepared from 2.7 g (0.0075 mol) of compound (a) and 3 g (0.0075 mol) of trans 1-(4-fluorophenyl)-hexahydrospiro[3H-oxazolo[3,4-a]pyridine-3,4'-piperidine] (disclosed in U.S. Patent No. 4,260,623) and 3 ml of triethylamine. The product is isolated as the monomaleate salt mp 171-173°C.

EXAMPLE V

N-[4-(2-Fluorobenzoyl)-1,3-dimethyl-1H-pyrazol-5-yl]-2-[[3-(2-methyl-1-piperidinyl)propyl]amino]acetamide, (Compound 20), a solution of 14.5 g (0.045 mol) of N-(4-bromo-1,3-dimethyl-1H-pyrazol-5-yl)-2-[[3-(2-methyl-1-piperidinyl)propyl]amino]acetamide in 150 ml of THF is cooled to -60 to -70°C by an acetone-dry ice bath and 105 ml (calc'd as 0.15 mol) of N-butyllithium in heptane is added dropwise under an atmosphere of nitrogen. The mixture is stirred at -65°C for 1.5 hours and a solution of 10 g (0.065 mol) of methyl o-fluorobenzoate in 20 ml THF is added dropwise. Stirring is continued 1 hour at -60°C, then the temperature is allowed to rise slowly to 0°C, when 70 ml of saturated ammonium chloride solution is added. The organic layer is separated, dried over $MgSO_4$ and evaporated in vacuo. The residue is dissolved in acetonitrile, treated with 2 equivalents of maleic acid, and the solution is diluted with ethyl acetate-ether to give the title compound as the dimaleate salt, mp 148-150°C.

EXAMPLE VI

N-[4-(2-Fluorobenzoyl)-1,3-dimethyl-1H-pyrazol-5-yl]-2-[[3-(2-methyl-1-piperidinyl)propyl]amino]acetamide (d(-)-form), (Compound 41), also the d(-)-form of compound 20. To 9.45 g (0.095 mol) of d(-)-2-methyl-1-piperidine [Chem. Ber., 29, 43 (1896)] in 50 ml of xylene is added 25.5 g (0.095 mol) of 1-bromo-3-phthalimidopropane. The reaction is refluxed overnight, cooled, filtered, and washed with xylene and hexane to give 35 g of crude d(-)(3-phthalimidopropyl)-2-methylpiperidine, mp 202.5-209° which is used without purificatin in the next step. To 35 g (0.095 mol) of d(-)-1-(3-phthalimdopropyl)-2-methyl-piperidine in 100 ml of ethanol is added 10 g (0.31 mol) of anhydrous hydrazine. The reaction is refluxed 0.5 hours during which time a flocculent white precipitate forms. The reaction is cooled and filtered and the filter cake is washed with ethyl ether. The combined filtrate is evaporated under vacuum. The residue and the filter cake are combined and slurried in ethyl ether. To the slurry is added 15 ml of 50% sodium hydroxide.

The resulting mixture is filtered and the filter cake is washed with ether. The two layers of the filtrate are separated and the ether layer is evaporated and distilled to give 10.3 g of d(-)-1-(3-aminopyropyl)-2-methylpiperidine, bp 86-87°C (13 mm), $[\alpha]_D^{23}$ - 67.1° (1% methanol).

To a solution of 9.78 g (0.0467 mol) of d(-)-1-(3-aminopropyl)-2-methylpiperidine in 180 ml toluene is added 8.28 g (.0234 mol) of compound (a). The resulting slurry is stirred at ambient temperature for three hours during which a clear solution is formed. The resulting solution is applied to the top of a column of about 250 g of silica gel which had been prewashed with a solution made up of 80 parts methylene chloride, 20 parts methanol, and 3 parts ammonium hydroxide. The column is developed with a solution containing 80 parts methylene chloride and 20 parts of methanol until the eluate begins to contain some of the starting piperidino compound. The combined product fractions are evaporated under vacuum and combined with a solution of 5.4 g of maleic acid in 40 ml of acetonitrile. The resulting solution is chilled and the precipitate is collected and washed with acetonitrile to give 11.5 g of N-[4-(2-fluorobenzoyl)-1,3-dimethyl-1H-pyrazol-5-yl]-2-[[3-(2-methyl-1-piperidinyl)-propyl]amino]acetamide (d(-)form); mp 126-130°C, $[\alpha]_D^{23}$ -6.4° (1% methanol), as the dimaleate hemihydrate.

<u>N-[4-(2-Fluorobenzoyl)-1,3-dimethyl-1H-pyrazol-5-yl]-2-[[3-(2-methyl-1-piperidinyl)propyl]amino]acetamide (l(+)-form)</u>, (Compound 42), also the l(+)-form of compound 20. A solution of 7.8 g (0.079 mol) of l(+)-2-methylpiperidine [Chem. Ber., <u>29</u>, 43(1896)] in 50 ml of xylene is reacted with 21.0 g of 1-bromo-3-phthalimidopropane to give 28.9 g of crude l(+)-(3-phthalimido-propyl)-2-methylpiperidine, mp 203-208.5°C which is used without purification in the next step. A solution of 28.9 g (0.078 mol) of l(+)-1-(3-phthalimidopropyl)-2-methylpiperidine in 100 ml of ethanol is reacted with 10.0 g (0.31 mol) of anhydrous hydrazine

to give 5.7 g of l(+)-1-(3-aminopropyl)-2-methylpiperidine, bp 86-87°C (13 mm),

$[\alpha]_D^{23}$ + 69.8° (1% methanol).

A solution of 5.2 g (0.025 mol) of l(+)-1-(3-aminopropyl)-2-methylpiperidine in 100 ml of toluene is reacted with 4.4 g (0.012 mol) of compound (a) to give 6.03 g of N-[4-(2-fluorobenzoyl)-1,3-dimethyl-1H-pyrazol-5-yl]-2-[[3-(2-methyl-1-piperidinyl)propyl]amino]acetamide (l(+)form), mp 132.5-134-5°C, as the dimaleate salt, hemihydrate,

$[\alpha]_D^{23}$ + 7.2° (1% methanol).

EXAMPLE VII

N-[4-(2-Fluorobenzoyl)-1,3-dimethyl-1H-pyrazol-5-yl]-2-[[3-(2-methyl-1-piperidinyl)propyl]thio]acetamide, (Compound 43), to a solution of 2.50 g (7 mmol) of compound (a) in 200 ml of toluene at 60°C is added all at once 2.44 g (14 mmol) of N-(3-thiopropyl)-2-methylpiperidine. The mixture is stirred at 60°C for 2 hours and at room temperature for 18 hours. The solution is washed with water and 5% sodium bicarbonate solution. The solvent is evaporated and the residue is recrystallized from hexane to afford 1.10 g of N-[4-(2-fluorobenzoyl)-1,3-dimethyl-1H-pyrazol-5-yl]-2-[[3-(2-methyl-1-piperidinyl)propyl]thio]acetamide, mp 90-91°C.

N-[4-(2-Fluorobenzoyl)-1,3-dimethyl-1H-pyrazol-5-yl]-2-[[2-(4-morpholinyl)ethyl]thio]acetamide, (Compound 44), from 7.39 g (20.9 mmol) of compound (a) and 6.14 g (41.8 mmol) of 2-(N-morpholino)ethanethiol and 60 ml of methylene chloride, there is obtained 3.34 g of N-[4-(2-fluorobenzoyl)-1,3-dimethyl-1H-pyrazol-5-yl]2(4-morpholinyl)ethyl]thio]acetamide, mp 87.5-89°C.

EXAMPLE VIII

N-[4-(2-Fluorobenzoyl)-1,3-dimethyl-1H-pyrazol-5-yl]-2-[[3-(2-methyl-1-piperidinyl)propyl]amino]acetamide, (Compound 20), A solution of 18.7 g (0.053 mol) of compound (a) in 1 L of methylene chloride is treated in one portion with 16.5 g (0.106 mol) of 1-(3-aminopropyl)-2-pipecoline and stirred at reflux for 17 hours. The mixture is cooled and washed two times with 50 ml of 5% sodium bicarbonate solution. The methylene chloride

solution is dried over magnesium sulfate and evaporated to dryness. The residue is taken up in 1 L of ether and filtered. The solution is treated with ethereal maleic acid. The precipitate is collected and recrystallized from acetonitrile-ethyl acetate to give 9.2 g of N-[4-(2-fluorobenzoyl)-1,3-dimethyl-1H-pyrazol-5-yl]-2-[(3-(2-methyl-1-piperidinyl)propyl)-amino]acetamide, dimaleate salt; mp 146-149°C.

The filtrate from the above recrystallization is chromatographed on a silica gel column. After recrystallization from ethyl acetate there is isolated 1.20 g of 4-(2-fluorophenyl)-1,3-dimethyl-5-[(3-(2-methyl-1-piperidinyl)propyl)amino]-1H-pyrazolo[3,4-_b_]pyridin-6-ol; mp 172-174°C, compound I'.

A solution of 1.0 g (2.43 mmol) of 4-(2-fluorophenyl-1,3-dimethyl-5-[(3-(2-methyl-1-piperidinyl)propyl)amino]-1H-pyrazolo[3,4-_b_]pyridin-6-ol in 100 ml of 2 N sodium hydroxide is stirred overnight at room temperature. The solution is neutralized to pH 7 with 2 N hydrochloric acid and evaporated to dryness. The product is taken up in methylene chloride and added to a solution of ethereal maleic acid. The resulting precipitate is collected and recrystallized from ethyl acetate-acetonitrile to give pure N-[4-(2-fluorobenzoyl-1,3-dimethyl-1H-pyrazol-5-yl]-2-[[3-(2-methyl-1-piperidinyl)propyl]amino]acetamide, dimaleate salt, compound 20.

## PREPARATIVE EXAMPLES

### I

N-[4-(2-Fluorobenzoyl)-1,3-dimethyl-1H-pyrazol-5-yl]-2-bromoacetamide, (Compound "a") A solution of 12 g (0.05 mol) of 5-amino-1,3-dimethyl-1H-pyrazol-4-yl-(2-fluorophenyl)methanone (U.S. Patent No. 3,558,605) in 150 ml of warm dichloroethane is treated dropwise with 10 g (0.05 mol) of bromoacetylbromide and stirred under reflux for 2-3 hours. The solution is cooled with an ice bath and 100 ml of a saturated solution of $NaHCO_3$ is added. A solid separates and is collected by filtering. After washing the filter cake with water and a little dichloroethane, the product, N-[4-(2-fluorobenzoyl)-1,3-dimethyl-1H-pyrazol-5-yl]-2-bromoacetamide is obtained in pure form as a cream colored solid; 14.4 g (82%), mp 195-197°C.

In a similar manner but starting with 5-amino-1,3-dimethyl-1H-pyrazol-4-yl(3-or 4-fluorophenyl)methanone there is obtained N-[4-(3-fluorobenzoyl)-1,3-dimethyl-1H-pyrazol-5-yl]-2-bromo-acetamide mp 185-187°C, recrystallized from ethyl acetate; and N-[4-(4-fluorobenzoyl)-1,3-dimethyl-1H-pyrazol-5-yl]-2-bromoacetamide mp 163-165°C, recrystallized from ethyl acetate/petroleum ether.

II

N-(4-Bromo-1,3-dimethyl-1H-pyrazol-5-yl)-2-[[3-(2-methyl-1-piperidinyl)propyl]amino]acetamide, To a solution of 22 g (0.2 mol) of 5-amino-1,3-dimethylpyrazole in 150 ml of refluxing dichloroethane is added dropwise 40 g (0.2 mol) of bromoacetylbromide. Refluxing is continued for 2 hours then stirred overnight at 25°C. The mixture is diluted with 200 ml of ether and the HBr salt of N-(1,3-dimethyl-1H-pyrazol-5-yl)-2-bromacetamide, mp 143-145°C, is collected by filtering.

A suspension of 16 g (0.05 mol) of this HBr salt in 200 ml of dichloromethane is stirred and treated with 15 ml (0.1+ mol) of triethylamine and 9 g (0.057 mol) of 1-(3-aminopropyl)-2-methylpiperidine. After stirring for 16-24 hours the solution is washed with 75 ml of conc. $NH_4OH$. The organic layer is separated, dried over $MgSO_4$ and evaporated to give 14 g of oil, which is characterized as the dimaleate salt, mp 164-166°C. To this product, N-[1,3-dimethyl-1H-pyrazol-5-yl]2-[[3-(2-methyl-1-piperidinyl)propyl]amino]acetamide, in 150 ml dichloromethane 14 g (0.05 mol) of bromine is added dropwise with stirring and cooling at 0-5°C. After stirring 1-2 hours, the mixture is stirred with 50 ml of conc. $NH_4OH$; the organic layer is separated, dried over $MgSO_4$ and evaporated in vacuo. The product, 15 g, N-(4-bromo-1,3-dimethyl-1H-pyrazol-5-yl)-2-[[3-(2-methyl-1-piperidinyl)propyl]amino]acetamide is characterized as the dimaleate salt, mp 180-182°C dec.

III

General process for (5-Amino-1,3-dialkyl-1H-pyrazol-4-yl)aroyl-methanones

(5-Amino-1,3-dimethyl-1H-pyrazol-4-yl)(2-fluorophenyl)methanone is obtained by treatment of N-[4-(2-fluorobenzoyl)-1,3-dimethyl-1H-pyrazol-5-yl]benzamide (105 g 0.31 mol) with 640 ml of 75% sulfuric acid (V/V) at 90-95°C for 5 hours [cf the procedure of U.S. Patent No. 3,660,425, Example 2-a]. The product (5-Amino-1,3-dimethyl-1H-pyrazol-4-yl)(2-fluorophenyl)methanone 63.5 g (88%) melts at 106-109°C after recrystallization from ether or toluene.

The requisite N-[(4-aroyl)-1,3-dialkyl-1H-pyrazol-5-yl]-benzamides are obtained as follows:

A solution of 118 g (0.4 mol) of N-(4-bromo-1,3-dimethyl-1H-pyrazol-5-yl)benzamide in 1.2 L of tetrahydrofuran is cooled to -60° t -70°C under nitrogen and 500 ml of Nbutyllithium in heptane (calc'd as 0.8 mol) is added dropwise with stirring during 0.5 hours. The resulting suspension is stirred at -60°C another hour, and then 62 g (0.4 mol) of methyl o-fluorobenzoate (or an equivalent of o-fluorobenzoyl chloride) is added dropwise. The mixture is stirred and allowed to warm slowly to -5° to 0°C during 1-2 hours, and 300 ml of a saturated solution of ammonium chloride is added. A new solid separates on continued stirring and is collected by filtering. The product is washed with water, then ether to give 106 g (80%), mp 220-222°C, of N-[4-(2-fluoro-benzoyl)-1,3-dimethyl-1H-pyrazol-5-yl]benzamide. In a similar manner, but using methyl m-fluorobenzoate or methyl p-fluorobenzoate (or an equivalent of the corresponding acid chloride) in place of methyl o-fluorobenzoate there is obtained N-[4-(3-fluorobenzoyl)-1,3-dimethyl-1H-pyrazol-5-yl]benzamide, mp 173-175°C, recrystallized from ethyl acetate; and N-[4-(4-fluoro-benzoyl)-1,3-dimethyl-1H-pyrazol-5-yl]benzamide, mp 205-207°C, recrystallized from ethyl acetate.

The requisite bromopyrazole is obtained as follows:

N-(1,3-dimethyl-1H-pyrazol-5-yl)benzamide (336 g, 1.56 mol), mp 133-4°C, is dissolved in 2 L of dichloromethane and with stirring, bromine (250 g, 1.56 mol) is added dropwise at 20-25°C. The resulting suspension of the hydrobromide salt of the product is stirred with excess 3N $NH_4OH$. The organic layer is separated, dried over $MgSO_4$ and concentrated in vacuo to a slurry. The slurry is stirred with 700 ml of petroleum ether and filtered to give 435 g (95%), mp 125-127°C, of N-(4-bromo-1,3-dimethyl-1H-pyrazol-5-yl)benzamide.

CLAIMS (for all designated states other than Austria):

1. A compound having the following structural formula:

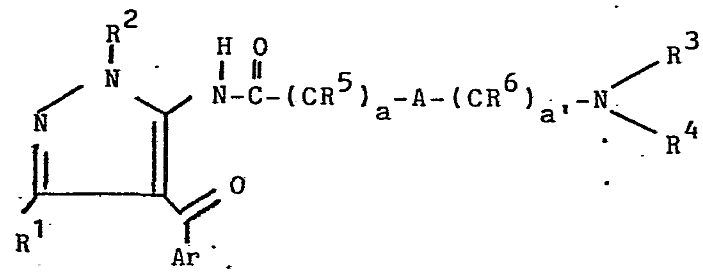

(I)

wherein each of $R^1$, $R^5$ and $R^6$, which are the same or different, is a hydrogen atom or an alkyl radical of from 1 to 6 carbon atoms; $R^2$ is an alkyl radical of from 1 to 6 carbon atoms; $R^3$ is a hydrogen atom, an alkyl radical of from 1 to 6 carbon atoms, a cycloalkyl radical of from 3 to 12 carbon atoms, or an aryl or alkaryl radical; and $R^4$ is a hydrogen atom, an alkyl radical of from 1 to 6 carbon atoms, a cycloalkyl radical of 3 to 12 carbon atoms, an aryl or alkaryl radical, a cycloalkene radical of from 5 to 7 carbon atoms which is optionally substituted with 1 or 2 alkyl radicals of from 1 to 3 carbon atoms, or a cycloalkenone radical of from 5 to 7 carbon atoms which is optionally substituted with 1 or 2 alkyl radicals of from 1 to 3 carbon atoms; or $R^3$ and $R^4$, when taken together with the nitrogen atom to which they are attached, either form a dihydropyridine, tetrahydropyridine, piperazine, piperidine, pyrrolidine or morpholine ring which is optionally substituted with 1 or 2 alkyl radicals of from 1 to 6 carbon atoms, a phenyl radical or a benzyl radical, or form one of the following substitutents:

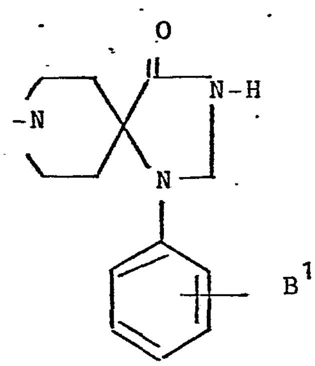

where $B^1$ is a hydrogen or halogen atom or a lower alkyl or lower alkoxy radical;

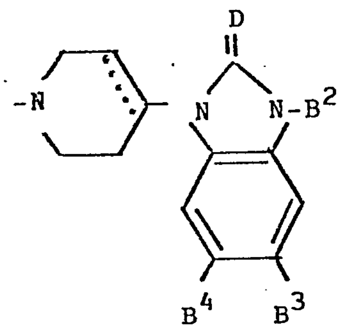

where the dotted line indicates an optional double bond; D is an oxygen or sulphur atom; $B^2$ is a hydrogen atom or a lower alkyl radical; and each of $B^3$ and $B^4$, which are the same or different, is a hydrogen or halogen atom or a lower alkyl, lower alkoxy or trifluoromethyl radical;

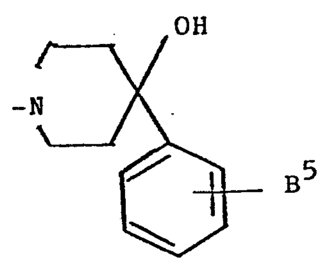

where $B^5$ is a hydrogen or halogen atom or a lower alkyl or trifluoromethyl radical;

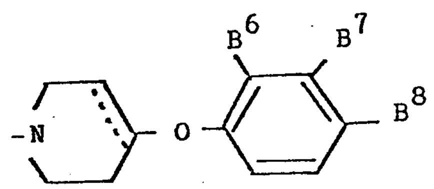

where the dotted line indicates an optional double bond; and each of $B^6$, $B^7$ and $B^8$, which are the same or different, is a hydrogen or halogen atom or a lower alkyl, lower alkoxy or trifluoromethyl radical;

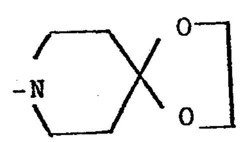

; or

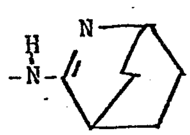

;

Ar is a phenyl radical; a phenyl radical substituted with a halogen atom, an alkyl radical of from 1 to 3 carbon atoms, an alkoxy radical of from 1 to 3 carbon atoms, or a trifluoromethyl radical; 2-, 3-, or 4-pyridyl

or 2- or 3-thienyl; A is a bond or the group $OCH_2CH_2$, $SCH_2CH_2$ or $NR^7$ wherein $R^7$ is a hydrogen atom or an alkyl radical of from 1 to 6 carbon atoms; and each of a and a', which are the same or different, is zero or an integer of 1 to 6, provided that, when a is 1, $R^5$ must be a hydrogen atom; or a pharmaceutically acceptable salt thereof.

2. A compound according to Claim 1, wherein both $R^1$ and $R^2$ are methyl radicals.

3. A compound according to Claim 1 or 2, wherein Ar is 2-fluorophenyl.

4. A compound according to Claim 1, which is N-[4-(2-fluorobenzoyl)-1,3-dimethyl-1H-pyrazol-5-yl]-1-pyrrolidineacetamide or a pharmaceutically acceptable salt thereof.

5. A compound according to Claim 1, which is 2-(diethyl-amino)-N-[4-(2-fluorobenzoyl)-1,3-dimethyl-1H-pyrazol-5-yl]-acetamide or a pharmaceutically acceptable salt thereof.

6. A compound according to Claim 1, which is N-[4-(2-fluorobenzoyl)-1,3-dimethyl-1H-pyrazol-5-yl]-3-[[(2-methyl-1,3-dioxolan-2-yl)methyl]amino]acetamide or a pharmaceutically acceptable salt thereof.

7. A compound according to Claim 1, which is N-[4-(2-fluorobenzoyl)-1,3-dimethyl-1H-pyrazol-5-yl]-2-(bicyclo-[2,2,1]hept-2-ylamino)acetamide or a pharmaceutically acceptable salt thereof.

8. A compound according to Claim 1, which is N-[4-(2-fluorobenzoyl-1,3-dimethyl-1H-pyrazol-5-yl]-4-oxo-1-phenyl-1,3,8-triazaspiro[4,5]decan-8-acetamide or a pharmaceutically acceptable salt thereof.

9. A compound according to Claim 1, which is 2-(diethyl-amino)-N-[1,3-dimethyl-4-(3-thienylcarbonyl)-1H-pyrazol-5-yl]- acetamide or a pharmaceutically acceptable salt thereof.

10. A compound according to Claim 1, which is 2-(diethyl-amino)-N-[1,3-dimethyl-4-(2-thienylcarbonyl)-1H-pyrazol-5-yl]-acetamide or a pharmaceutically acceptable salt thereof.

11. A compound according to Claim 1, which is N-[4-(2-fluorobenzoyl)-1,3-dimethyl-1H-pyrazol-5-yl]-2-[[3-(2-methyl-1-piperidinyl)propyl]amino]acetamide or a pharmaceutically acceptable salt thereof.

12. A compound according to Claim 1, which is N-[4-(2-fluorobenzoyl)-1,3-dimethyl-1H-pyrazol-5-yl]-2-[[3-(t-butylamino)-propyl]amino]acetamide or a pharmaceutically acceptable salt thereof.

13. A compound according to Claim 1, which is N-[4-(2-fluorobenzoyl)-1,3-dimethyl-1H-pyrazol-5-yl]-2-[[3-(2-methyl-1-piperidinyl)propyl]thio]acetamide or a pharmaceutically acceptable salt thereof.

14. A compound having the following structural formula:

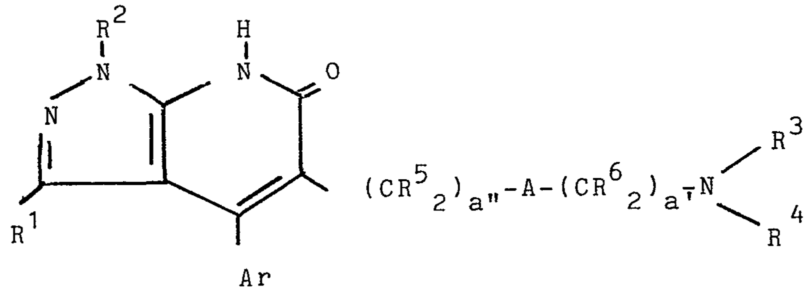

wherein $R^1$, $R^2$, $R^3$, $R^4$, $R^5$, $R^6$, Ar, A and a' are as defined in Claim 1, and a" is zero or an integer of 1 to 5, or a pharamceutically acceptable salt thereof.

15. A compound according to Claim 14, which is 4-(2-fluorophenyl-1,3-dimethyl-5-[(3-(2-methyl-1-piperidinyl)propyl)amino]-1H-pyrazolo[3,4-b]pyridin-6-ol or a pharmaceutically acceptable salt thereof.

16. A process for producing a compound having the formula I defined in Claim 1, which process comprises reacting a compound having the following structural formula

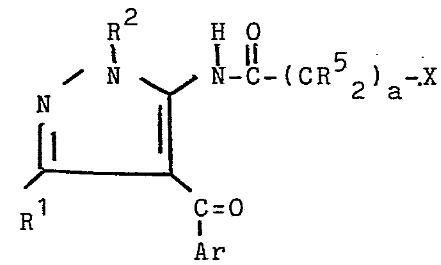

with a compound having the following structural formula

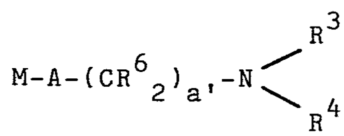

wherein $R^1$, $R^2$, $R^3$, $R^4$, $R^5$, $R^6$, Ar, A, a and a' are as defined in Claim 1, M is a hydrogen atom or an alkali metal atom, and X is a halogen atom.

17. A process for producing a compound having the formula I defined in Claim 1, which process comprises reacting a compound having the following structural formula

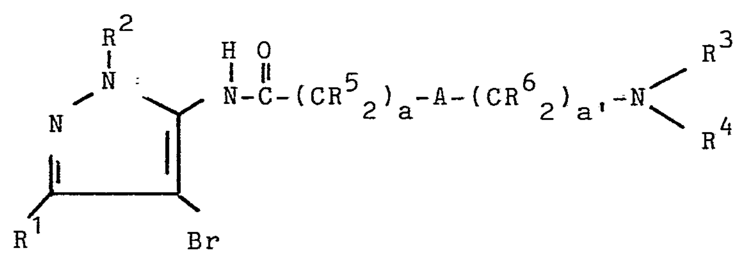

with a compound having the following structural formula

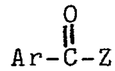

wherein $R^1$, $R^2$, $R^3$, $R^4$, $R^5$, $R^6$, Ar, A, a and a' are as defined in Claim 1 and Z is a halogen atom, an alkoxy radical or an equivalent thereof capable of reacting with the bromine atom.

18. A process for producing a compound having the formula I defined in Claim 1 wherein the carbon alpha to the amide carbonyl group is part of a methylene radical, which process comprises treating with a base a compound having the following structural formula

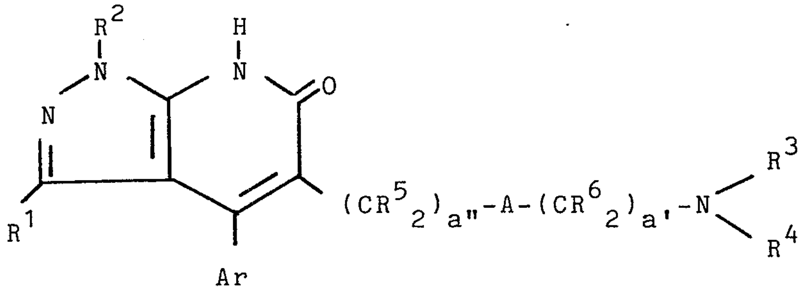

wherein $R^1$, $R^2$, $R^3$, $R^4$, $R^5$, $R^6$, Ar, A and a' are as defined in Claim 1 and a" is zero or an integer from 1 to 5.

19. A pharmaceutical composition suitable for use in treating psychoses, which composition comprises one or more compound as claimed in Claim 1, in combination with a pharmaceutically acceptable carrier.

20. A process for producing a compound having the formula I' defined in Claim 14, which process comprises reacting a compound having the following structural formula

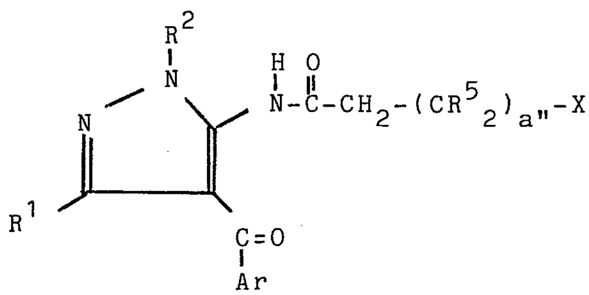

with a compound having the following structural formula

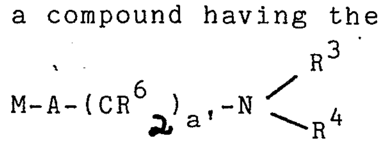

wherein $R^1$, $R^2$, $R^3$, $R^4$, $R^5$, $R^6$, Ar, A and a' are as defined in Claim 1, a" is zero or an integer of from 1 to 5, M is a hydrogen atom or an alkali metal atom, and X is a halogen atom.

CLAIMS (for Austria alone):

1. A process for producing a compound having the following structural formula:

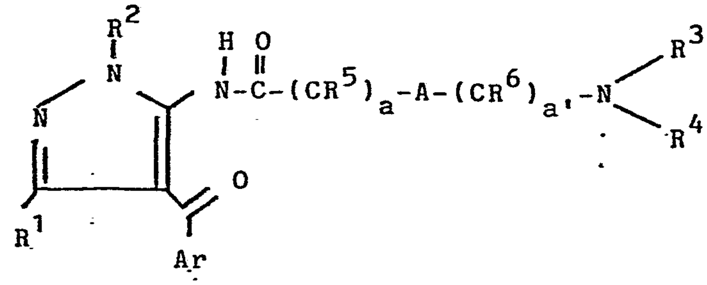

(I)

wherein each of $R^1$, $R^5$ and $R^6$, which are the same or different, is a hydrogen atom or an alkyl radical of from 1 to 6 carbon atoms ; $R^2$ is an alkyl radical of from 1 to 6 carbon atoms; $R^3$ is a hydrogen atom, an alkyl radical of from 1 to 6 carbon atoms, a cycloalkyl radical of from 3 to 12 carbon atoms, or an aryl or alkaryl radical; and $R^4$ is a hydrogen atom, an alkyl radical of from 1 to 6 carbon atoms, a cycloalkyl radical of 3 to 12 carbon atoms, an aryl or alkaryl radical, a cycloalkene radical of from 5 to 7 carbon atoms which is optionally substituted with 1 or 2 alkyl radicals of from 1 to 3 carbon atoms, or a cycloalkenone radical of from 5 to 7 carbon atoms which is optionally substituted with 1 or 2 alkyl radicals of from 1 to 3 carbon atoms; or $R^3$ and $R^4$, when taken together with the nitrogen atom to which they are attached, either form a dihydropyridine, tetrahydropyridine, piperazine, piperidine, pyrrolidine or morpholine ring which is optionally substituted with 1 or 2 alkyl radicals of from 1 to 6 carbon atoms, a phenyl radical or a benzyl radical, or form one of the following substitutents:

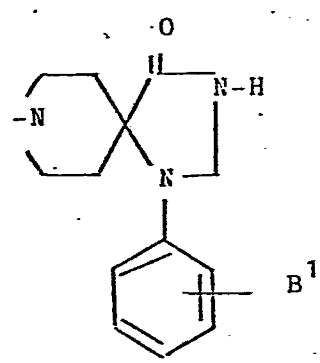

where $B^1$ is a hydrogen or halogen atom or a lower alkyl or lower alkoxy radical;

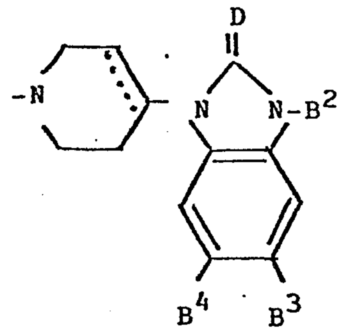

where the dotted line indicates an optional double bond; D is an oxygen or sulphur atom; $B^2$ is a hydrogen atom or a lower alkyl radical; and each of $B^3$ and $B^4$, which are the same or different, is a hydrogen or halogen atom or a lower alkyl, lower alkoxy or trifluoromethyl radical;

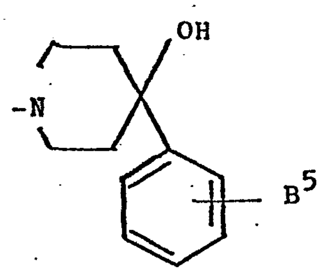

where $B^5$ is a hydrogen or halogen atom or a lower alkyl or trifluoromethyl radical;

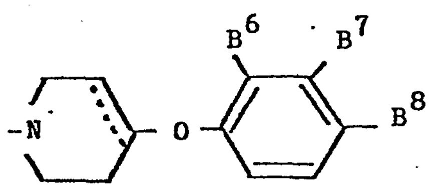

where the dotted line indicates an optional double bond; and each of $B^6$, $B^7$ and $B^8$, which are the same or different, is a hydrogen or halogen atom or a lower alkyl, lower alkoxy or trifluoromethyl radical;

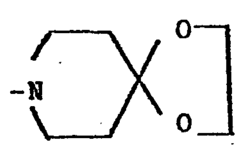

; or

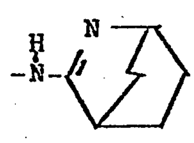

;

Ar is a phenyl radical; a phenyl radical substituted with a halogen atom, an alkyl radical of from 1 to 3 carbon atoms, an alkoxy radical of from 1 to 3 carbon atoms, or a trifluoromethyl radical; 2-, 3-, or 4-pyridyl

or 2- or 3-thienyl; A is a bond or the group $OCH_2CH_2$, $SCH_2CH_2$ or $NR^7$ wherein $R^7$ is a hydrogen atom or an alkyl radical of from 1 to 6 carbon atoms; and each of a and a', which are the same or different, is zero or an integer of 1 to 6, provided that, when a is 1, $R^5$ must be a hydrogen atom; or a pharmaceutically acceptable salt thereof; which process comprises reacting a compound having the following structural formula

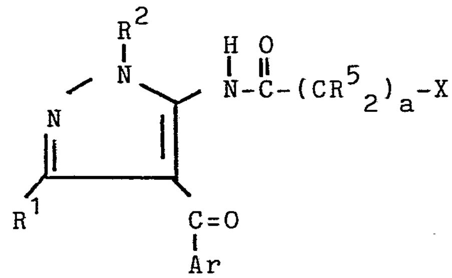

with a compound having the following structural formula

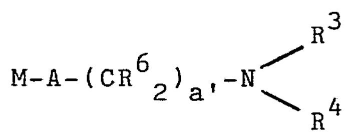

wherein $R^1$, $R^2$, $R^3$, $R^4$, $R^5$, $R^6$, Ar, A, a and a' are as defined above, M is a hydrogen atom or an alkali metal atom, and X is a halogen atom; and, if necessary, converting the reaction product to a pharmaceutically acceptable salt.

2. A process for producing a compound of formula I as defined in Claim 1, which process comprises reacting a compound having the following structural formula

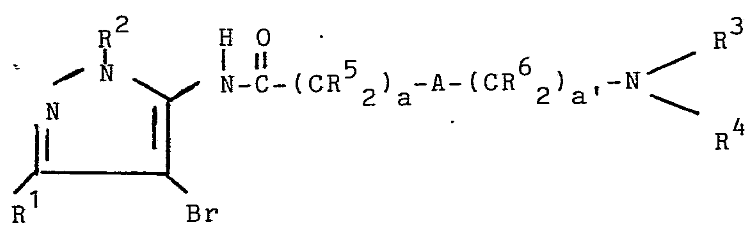

with a compound having the following structural formula

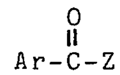

wherein $R^1$, $R^2$, $R^3$, $R^4$, $R^5$, $R^6$, Ar, A, a and a' are as defined in Claim 1, and Z is a halogen atom, an alkoxy radical or an equivalent thereof capable of reacting with the bromine atom; and, if necessary, converting the reaction product to a pharmaceutically acceptable salt.

3. A process for producing a compound having the formula I defined in Claim 1 wherein the carbon atom alpha to the amide carbonyl group is part of a methylene group, which process comprises treating with a base a compound having the following structural formula

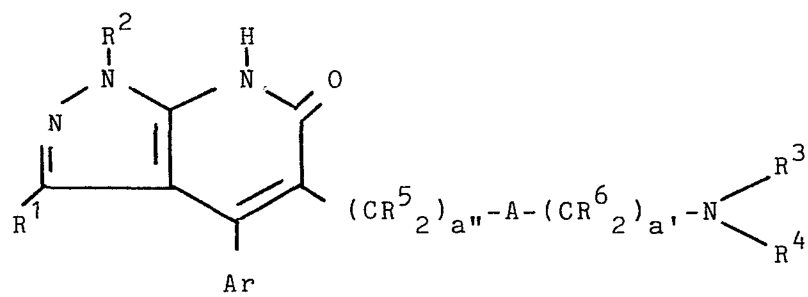

wherein $R^1$, $R^2$, $R^3$, $R^4$, $R^5$, $R^6$, Ar, A, and a' are as defined in Claim 1 and a" is zero or an integer from 1 to 5; and, if necessary, converting the product of the treatment to a pharmaceutically acceptable salt.

4. A process for producing a pharmaceutical composition useful for treating psychoses, which comprises admixing a compound of formula I as defined in Claim 1 or mixtures thereof, with a pharmaceutically acceptable carrier.

5. A process for producing a compound having the following general formula:

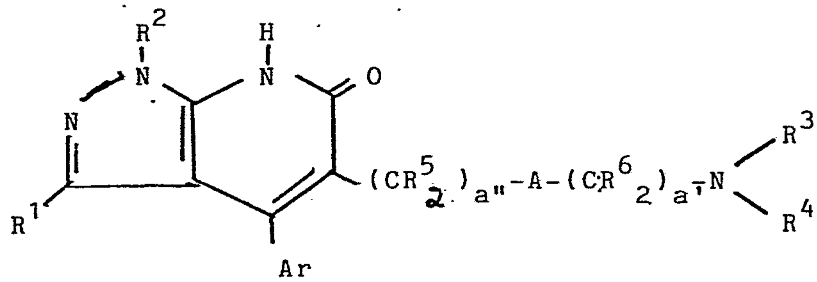

which process comprises reacting a compound having the following structural formula

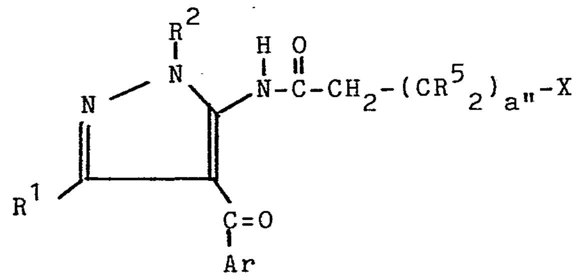

with a compound having the following structural formula:

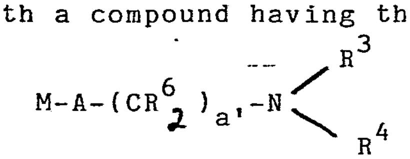

wherein $R^1$, $R^2$, $R^3$, $R^4$, $R^5$, $R^6$, Ar, A and a' are as defined in Claim 1, a" is zero or an integer of from 1 to 5, M is a hydrogen atom or an alkali metal atom, and X is a halogen atom.